(19) Europäisches Patentamt
European Patent Office
Office européen des brevets

(11) Veröffentlichungsnummer: **0 108 295**
**A1**

(12) # EUROPÄISCHE PATENTANMELDUNG

(21) Anmeldenummer: 83110343.7

(22) Anmeldetag: 17.10.83

(51) Int. Cl.³: A 61 K 9/00
A 61 K 47/00, A 61 K 31/43
A 61 K 31/545, A 61 K 31/40
A 61 K 31/535

(30) Priorität: 15.10.82 US 434452

(43) Veröffentlichungstag der Anmeldung: 16.05.84 Patentblatt 84/20

(84) Benannte Vertragsstaaten: AT BE CH DE FR GB IT LI LU NL SE

(71) Anmelder: F. HOFFMANN-LA ROCHE & CO. Aktiengesellschaft

CH-4002 Basel(CH)

(72) Erfinder: Behl, Charanjit
280 Hoover Avenue
Bloomfield, N.J.(US)

(72) Erfinder: Beskid, George
565 Park Street
Upper Montclair, N.J.(US)

(72) Erfinder: Shah, Navnit
203 Beverly Hill Road
Clifton, N.J.(US)

(72) Erfinder: Tossounian, Jacques
135 Konner Avenue
Pine Brook, N.J.(US)

(72) Erfinder: Unowsky, Joel
37 Fellswood Drive
Livingston, N.J.(US)

(74) Vertreter: Lederer, Franz, Dr. et al,
Patentanwälte Dr. Franz Lederer Dipl.-Ing. Reiner F. Meyer-Roxlau Lucile-Grahn-Strasse 22
D-8000 München 80(DE)

(54) **Orale Dosierungsform.**

(57) Die Erfindung betrifft ein absorbierbares pharmazeutisches Präparat für enterale Verabreichung, dadurch gekennzeichnet, dass es eine therapeutisch wirksame Menge eines im wesentlichen oral inaktiven β-Lactam-Antibiotikums in einer absorptionserhöhenden Menge eines Potentiators enthält, der aus einer aliphatischen $C_2$-$C_{18}$-Fettsäure, einem Mono-, Di- oder Triglycerid einer $C_2$-$C_{12}$-Fettsäure, einem partiellen Ester oder Vollester von Propylenglykol, Polyäthylenglykol oder einem Kohlenhydrat mit einer $C_2$-$C_{12}$-Fettsäure, einem pharmazeutisch verträglichen Ester bzw. Aether davon oder einem Gemisch der erwähnten Potentiatoren besteht; erwünschtenfalls in Verbindung mit einem pharmazeutisch verträglichen Träger. Die Erfindung betrifft auch die Herstellung solcher Präparate. Die Präparate sind für die Bekämpfung bzw. Verhütung von Infektionskrankheiten verwendbar.

EP 0 108 295 A1

Croydon Printing Company Ltd.

0108295

F.HOFFMANN-LA ROCHE & CO. Aktiengesellschaft, Basel/Schweiz

17. ... 1983

RAN 4410/169

# Orale Dosierungsform

Die vorliegende Erfindung betrifft ein absorbierbares pharmazeutisches Präparat, wobei im wesentlichen oral inaktive β-Lactam-Antibiotika (einschliesslich ihre pharmazeutisch verträgliche Salze und ihre Ester, Aether, und Hydrate bzw. Hydrate der Salze) in enteral oder oral wirksame Produkte übergeführt werden. Das Präparat enthält eine Kombination des gewählten β-Lactam-Antibiotikums mit einem bestimmten Potentiator. Die Erfindung betrifft insbesondere ein absorbierbares Präparat für enterale Verabreichung, dadurch gekennzeichnet, dass es eine therapeutisch wirksame Menge eines im wesentlichen oral inaktiven β-Lactam-Antibiotikums in einer absorptionserhöhenden Menge eines Potentiators enthält, der aus einer aliphatischen $C_2$-$C_{18}$-Fettsäure, einem Mono-, Di- oder Triglycerid einer $C_2$-$C_{12}$-Fettsäure, einem partiellen Ester oder Vollester von Propylenglykol, Polyäthylenglykol oder einem Kohlenhydrat mit einer $C_2$-$C_{12}$-Fettsäure, einem pharmazeutisch verträglichen Ester bzw. Aether davon oder einem Gemisch der erwähnten Potentiatoren besteht; erwünschtenfalls in Verbindung mit einem pharmazeutisch verträglichen Träger. Das Präparat liegt vorzugsweise in "enteric coated" Form vor, in welchem Falle es oral wirksam ist und für die orale Verabreichung verwendet werden kann.

Mn/19.9.83

Die Definition "enteric coated" Form bedeutet, dass das Präparat mit einem magensaftresistenten Ueberzug versehen ist, entweder die Dosierungsform selbst, z.B. die Kapsel oder Tablette, oder auch die beiden Komponenten, d.h. das β-Lactam-Antibiotikum bzw. der Potentiator, wie nachstehend näher erläutert wird.

Die Erfindung betrifft ebenfalls ein Verfahren zur Ueberführung von im wesentlichen oral inaktiven β-Lactam-Antibiotika in enteral wirksame Produkte, dadurch gekennzeichnet, dass man das erwähnte Antibiotikum mit einer absorptionserhöhenden Menge eines Potentiators kombiniert, der aus einer aliphatischen $C_2$-$C_{18}$-Fettsäure, einem Mono-, Di- oder Triglycerid einer $C_2$-$C_{12}$-Fettsäure, einem partiellen oder totalen Ester von Propylenglykol, Polyäthylenglykol oder einem Kohlenhydrat mit einer $C_2$-$C_{12}$-Fettsäure, einem pharmazeutisch verträglichen Ester bzw. Aether davon oder einem Gemisch der erwähnten Potentiatoren besteht; und erwünschtenfalls das Kombinationsprodukt mit einem pharmazeutisch verträglichen Träger vereinigt.

Wie gesagt, wird das Kombinationsprodukt vorzugsweise in "enteric coated" Form umgewandelt, wobei das erhaltene Produkt oral wirksam ist und für die orale Verabreichung verwendet werden kann.

Bevorzugte Potentiatoren sind gesättigte $C_6$-$C_{12}$-Fettsäuren, ungesättigte $C_{16}$-$C_{18}$-Fettsäuren, Mono-, Di- oder Triglyceride von $C_8$-$C_{12}$-Fettsäuren oder Mischungen davon, Speiseöle mit den erwähnten Potentiatoren sowie Mischungen davon; die bevorzugten Potentiatoren sind Mono- und Diglycerid-Mischungen von $C_8/C_{10}$ gesättigten Fettsäuren mit einem vorwiegenden Anteil an Monoglycerid. Der Glyceridgemisch-Anteil liegt vorzugsweise im Bereich zwischen etwa 33 und etwa 98 Gew.-% des Präparates, vorzugsweise bei etwa 89 Gew.-% des Präparates.

Die britische Patentschrift Nr. 1,432,784 offenbart

eine Methode zur oralen Verabreichung durch Kombination eines oral wirksamen Arzneimittels, z.B. eines Antibiotikums, mit einem Monoglycerid oder einem Triglycerid einer $C_6$-$C_{12}$-Fettsäure, welche eine erhöhte orale Absorption bewirkt. Es war im Lichte dieses Standes der Technik unerwartet, dass die Kombination eines oral wirksamen β-Lactam-Antibiotikums mit einem Potentiator, z.B. CAPMUL 8210 (einem Mono- und Diglycerid-Gemisch einer $C_8/C_{10}$ gesättigten Fettsäure) keine Wirkungssteigerung (d.h. keine verbesserte gastrointestinale Absorption) bewirkte, wogegen die Kombination eines oral inaktiven β-Lactam-Antibiotikums mit CAPMUL 8210 oder CAPMUL MCM90 in "enteric coated" Dosierungsform nach oraler Verabreichung therapeutisch signifikante Antibiotika-Blutspiegel zeigte. Weitere Versuche mit Kombinationen anderer β-Lactam-Antibiotika und Mischungen von Mono-, Di- und Triglyceriden, ihren pharmazeutisch verträglichen Salzen, Estern und Aethern und den Fettsäuren bestätigten diesen Effekt, wie nachstehend gezeigt wird.

Die belgische Patentschrift Nr. 567,598 offenbart eine orale (als Suspension) oder parenterale Methode zur Verabreichung durch Kombination eines Antibiotikums, z.B. eines Penicillins, mit einem Fettsäureglyceridgemisch, vorzugsweise einem Triglyceridgemisch. Erfindungsgemäss wurde jedoch gefunden, dass unerwartet hohe Antibiotikablutspiegel erreicht werden können durch Verwendung eines "enteric" Systems in Verbindung mit der Antibiotikum/Potentiator-Kombination. Dieser Befund ist wesentlich, denn es wurde ebenfalls gefunden, dass therapeutisch unannehmbare, niedrige Antibiotika-Blutspiegel erhalten werden, wenn man die β-Lactam-Antibiotikum/Potentiator-Kombination als Lösung/Suspension verabreicht, wie nachstehend gezeigt wird.

Der Ausdruck "β-Lactam-Antibiotika" bedeutet Verbindungen mit einem β-Lactamring als zentrales Strukturelement, d.h. dem Strukturelement

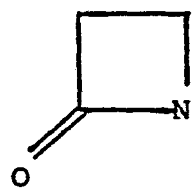

welches in verschiedenen Ringpositionen substituiert und/ oder mit anderen Ringsystemen, die selbst substituiert oder unsubstituiert sein können, kondensiert sein kann. Einige Beispiele bekannter β-Lactam-Antibiotika sind Penicilline, Cephalosporine, z.B. Ceftriaxon, monocyclische β-Lactame, z.B. Azthreonam; Thienamycin und seine Derivate sowie die Clavulansäurederivate, einschliesslich die pharmazeutisch verträglichen Salze der erwähnten Verbindungen.

Der Potentiator ist z.B. eine $C_2$-$C_{18}$ geradkettige oder verzweigtkettige bzw. gesättigte oder ungesättigte Fettsäure. Beispiele solcher Fettsäuren sind Buttersäure, Capronsäure, Caprylsäure, Caprinsäure, Laurinsäure, Myristinsäure, Palmitinsäure, Stearinsäure, Lauroleinsäure (Δ9-Dodecylensäure), Palmitoleinsäure, Oelsäure, Ricinolsäure, Linolsäure, Linolensäure, etc. Bevorzugte Potentiatoren sind $C_2$-$C_{12}$ geradkettige oder verzweigtkettige, gesättigte oder ungesättigte Mono-, Di- oder Triglyceride oder Mischungen davon, partielle Ester oder Vollester von Propylenglykol, Polyäthylenglykol und Kohlenhydraten mit $C_2$-$C_{12}$-Fettsäuren sowie pharmazeutisch verträgliche Ester oder Aether dieser Mono- oder Diglyceride.

Der Ausdruck "Fettsäuren" stellt eine Gruppe gesättigter oder ungesättigter, monobasischer aliphatischer Carbonsäuren dar, welche mit Glycerin oder anderen Alkoholen Ester bilden, wobei Fette, Oele, Wachse und andere Lipide entstehen.

Der Ausdruck "Glyceride" bezieht sich auf Ester von Glycerin, einschliesslich Fette und Oele, worin bis auf drei Moleküle Fettsäure mit einem Molekül Glycerin gebunden sind. Obwohl mehrheitlich die gleiche Fettsäure verwendet wird, können Glyceride von gemischten Fettsäuren verwendet werden. Bei Verwendung von solchen Glyceriden gemischter Fettsäuren können optisch aktive Verbindungen auftreten, welche ebenfalls einen Teil der vorliegenden Erfindung darstellen.

Als Potentiatoren kommen ebenfalls die Ester und Aether der erwähnten Mono- und Diglyceride bzw. der erwähnten partiellen Ester in Frage. Solche Ester und Aether können durch die Formeln

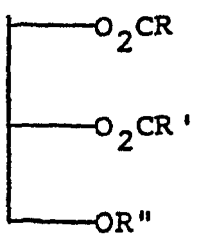 ; 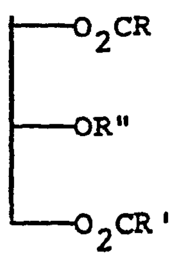 ; 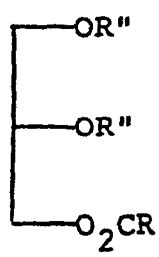 und 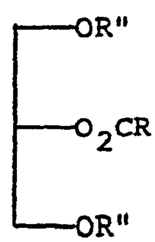

worin R und R' $C_2$-$C_{12}$-Fettsäurereste und gleiche oder verschiedene Bedeutungen haben können und R"O eine Ester- oder Aethergruppe darstellt,

dargestellt werden.

Geeignete Veresterungsagenzien sind beispielsweise solche, die aus pharmazeutisch verträglichen schwachen Säuren gebildet werden, wie Weinsäure und ihre Diacetylderivate, Essigsäure, Ascorbinsäure und Zitronensäure oder Phosphorsäuren mit einer Monophosphatgruppe, die einen Monophosphatester bilden kann.

Geeignete Aether können gebildet werden durch Umsetzung der Mono- oder Dihydroxyfunktion bzw. des Mono- oder Diglycerids mit einer reaktionsfähigen niederen Alkyl-, Alkenyl-, Alkinyl-, Aryl- oder substituierten Arylverbindung, wobei der entsprechende, pharmazeutisch verträgliche Äther entsteht. Diese Umsetzung erfolgt in an sich bekannter Weise.

Zwei Versuchsmethoden zur Ermittlung verwendbarer Potentiatoren, d.h. ein vivo und ein in vitro Versuch, werden nachstehend beschrieben.

In vivo Versuch

250 g schwere Ratten werden 20 mg/kg des β-Lactam-Antibiotikums intravenös, oral und enteral verabreicht. Für die orale Verabreichung werden die Antibiotika in destilliertem Wasser vorgelegt bzw. in einem der oben beschriebenen Potentiatoren, z.B. CAPMUL 8210 oder CAPMUL MCM90 (Warenzeichen von Stokely-Van Camp Inc., Columbus, Ohio), d.h. Mischungen von Mono- und Diglyceriden mit $C_8/C_{10}$ gesättigten Fettsäuren mit 70 bzw. 90% Monoglycerid, und zu 0,025 bis 1,0 ml verabreicht. Als Kontrollen wurden intravenöse Verabreichungen zu 0,5 ml durchgeführt. Für die enterale Verabreichung wurde das Antibiotikum in destilliertem Wasser bzw. in einem der oben beschriebenen Potentiatoren als Vehikel eingesetzt. Diese Mischungen wurden zu 0,025 bis 1,0 ml in das Duodenum unmittelbar unterhalb des Pylorus verabreicht. Suspensionen mit 20 mg Ceftriaxon in 100 µl CAPMUL 8210 oder CAPMUL MCM90 in "enteric coated" Hartgelatinekapseln wurden ebenfalls Totenkopfäffchen von 0,5-1 kg oral verabreicht. Dieser Tierspezies wurde ebenfalls in 1,0 ml Wasser intravenös und enteral sowie auch, in CAPMUL 8210 oder CAPMUL MCM90 dispergiert, enteral und oral verabreicht.

Die Plasmaspiegel der in obiger Weise hergestellten Präparate nach den verschiedenen Arten der Verabreichung wurden anfänglich durch Kardialpunktur der Ratten mit Hilfe einer heparinisierten Spritze gemessen. Anschliessend wurden drei Ratten entweder getötet und ausgeblutet, oder es wurden in verschiedenen Zeitabständen am Schwanz Blutproben entnommen; die Blutproben wurden vereinigt und

sofort zentrifugiert. Den Totenkopfäffchen wurden einzeln aus Vena bzw. Arteria femoralis mit Hilfe einer heparinisierten Spritze Blutproben entnommen. Die Antibiotika enthaltenden Blutproben wurden anhand mikrobiologischer "Grossplatten"-Analyse gemäss Lees und Tootill, Analyst 80, Seiten 95, 110 und 531 (1955) untersucht. Im Falle von Ceftriaxon wurde die Probe vor der Analyse durch Zusatz von Acetonitril entproteinisiert. Als Mikroorganismus zur Feststellung der Plasmaspiegel an Ceftriaxon wurde E. coli 1346 verwendet.

Die Ergebnisse der Blutspiegel an Ceftriaxon (µg/ml aus 250 g Ratten) in verschiedenen Zeitabständen nach einer Dosis von 20 mg des Antibiotikums (in Wasser bzw. in Potentiator) pro kg Körpergewicht werden in den nachstehenden Tabellen festgehalten.

Die erste Tabelle zeigt an der Ratte die Blutspiegel an Ceftriaxon (Säureform) und ebenfalls die signifikant grösseren Blutspiegel bei enteraler Verabreichung des Antibiotikums in CAPMUL 8210 im Vergleich zur enteralen Verabreichung des Antibiotikums in Wasser. Die zweite Tabelle zeigt an der Ratte die Blutspiegel nach Verabreichung des Natriumsalzes von Ceftriaxon intravenös in Wasser und wiederum die erheblich grösseren Blutspiegel nach enteraler Verabreichung des Antibiotikums in CAPMUL 8210 im Vergleich zur enteralen Verabreichung in Wasser. Auch nach oraler Verabreichung des Natriumsalzes von Ceftriaxon in Suspension mit CAPMUL 8210 wurden Blutspiegel, obwohl unannehmbar niedrig, erhalten. Dies entspricht den Angaben der oben erwähnten belgischen Patentschrift Nr. 567,598. Die dritte Tabelle zeigt an der Ratte die Ergebnisse der enteralen Absorption des Natriumsalzes von Ceftriaxon in Suspension mit anderen Glycerid-Potentiatoren, einschliesslich CAPMUL MCM90 (ähnliche Zusammensetzung wie CAPMUL 8210, jedoch mit etwa 90% Monoglyceriden im Vergleich zu den 70% in CAPMUL 8210).

Die vierte Tabelle zeigt die Blutspiegel des Natriumsalzes von Ceftriaxon in einer zweiten Spezies, dem Totenkopfäffchen. Die Blutspiegel sind angegeben für das Natriumsalz von Ceftriaxon nach intravenöser Verabreichung in Wasser, nach enteraler Verabreichung in CAPMUL 8210 oder Wasser, sowie nach oraler Verabreichung in Suspension mit CAPMUL 8210 und auch in "enteric coated" Hartgelatinekapseln mit 20 mg Natriumsalz von Ceftriaxon in Suspension mit 100 µg CAPMUL 8210. Aehnlich wie an der Ratte wurden nach enteraler Verabreichung des Natriumsalzes von Ceftriaxon in CAPMUL 8210 am Affen bedeutende Blutspiegel erreicht, jedoch nicht wenn die Verabreichung des Antibiotikums in Wasser erfolgte. Nach oraler Verabreichung des Antibiotikums in Suspension mit CAPMUL 8210 wurden nur niedrige Blutspiegel erreicht. Nach Verabreichung des Natriumsalzes von Ceftriaxon und CAPMUL 8210 in "enteric coated" Kapseln an drei Affen wurden gute Blutspiegel bei allen Affen beobachtet.

## Tabelle 1

Blutspiegel an Ceftriaxon-Säure - Ratten - µg/ml:

| Verabreichungsart<br>Träger | Enteral<br>CAPMUL 8210* | Enteral<br>$H_2O$ |
|---|---|---|
| Zeit (Min.) | | |
| 5 | 9 | <0,25 |
| 10 | 22 | 3 |
| 20 | 41 | <0,25 |
| 40 | 21 | 6 |
| 60 | 12 | <0,25 |
| 120 | 20 | <0,25 |

* Ein Gemisch von Mono- und Diglyceriden gesättigter $C_8/C_{10}$-Fettsäuren mit den folgenden Kenndaten:

| | Eigenschaft | Grenze | Testmethode |
|---|---|---|---|
| Spezifikation: | | | |
| | Jodwert | 1,5 max. | AOCS Cd 1-25 |
| | Farbe, Lovibond rot | 2,5 max. | AOCS Cd 13b-45 |
| | Säurewert | 2,5 max. | AOCS Cd 3a-63 |
| | Feuchtigkeit | 0,5% max. | AOCS Ca 2e-55 |
| | Monoglyceride (α, als Oleat) | 70% min. | AOCS Cd 11-57 |
| | freies Glycerin | 2,5% max. | USP |
| | Rückstand nach Verbrennung | 0,5% max. | USP |
| | Schwermetalle, als Blei | 10 ppm max. | USP |
| Typische Eigenschaften: | | | |
| | Spezifisches Gewicht (100°C) | 0,98-1,01 | USP |
| | Erscheinung | klare Flüssigkeit | visuell |
| | Viskosität | 40-55 cs | |

Abkürzungen: AOCS = American Oil Chemists' Society (Analysenmethoden)

USP = US Pharmacopeia (Analysenmethoden)

## Tabelle 2

**Blutspiegel an Ceftriaxon-Natriumsalz - Ratten - µg/ml**

| Verabreichungsart | i.V. | Enteral | | | Enteral | Oral |
|---|---|---|---|---|---|---|
| Träger | $H_2O$ | CAPMUL 8210 | | | $H_2O$ | CAPMUL 8210 |
| Zeit (Min.) | | | | | | |
| 5 | - | 12[1] | 12[2] | 39[3] | - | 6 |
| 10 | 132 | 26 | 23 | 44 | - | 5 |
| 20 | 112 | 40 | 31 | 21 | <0,25 | <0,25 |
| 40 | 79 | 12 | 25 | 8 | - | <0,25 |
| 60 | 72 | 52 | 26 | 7 | <0,25 | 6 |
| 120 | 25 | 18 | 18 | 6 | <0,25 | 4 |
| 180 | 12 | - | - | - | - | - |
| 240 | 3,0 | - | 12 | - | 0 | - |
| 360 | <0,25 | - | 8 | - | 0 | - |
| 480 | 0 | - | - | - | - | - |

[1] Zu jedem Zeitpunkt wurden jeweils drei Ratten getötet und ausgeblutet; die Plasmaproben wurden vereinigt.

[2] Zu jedem Zeitpunkt wurde jeweils an drei Ratten eine Schwanzblutprobe entnommen; die Plasmaproben wurden vereinigt.

[3] 25 µl CAPMUL 8210 Träger (sämtliche verbleibenden Kolonnen beziehen sich auf 500 µl) enthielten 5 mg Ceftriaxon.

Tabelle 3

Blutspiegel an Ceftriaxon-Natriumsalz in verschiedenen Trägern - Ratten - µg/ml

| Verabreichungsart Träger | Enteral MCM90[1] | Enteral Diacetin[2] | Enteral Monoacetin[3] | Enteral Triacetin[4] | Enteral Glyceryl-Monolaurat[5] | Enteral CAPREX 300[6] |
|---|---|---|---|---|---|---|
| Zeit (Min.) | | | | | | |
| 5 | 31 | 12 | 17 | 0,25 | 23 | 0 |
| 10 | 37 | 70 | 6 | 15 | 23 | 24 |
| 20 | 38 | 32 | 46 | 16 | 13 | 10 |
| 40 | 49 | 56 | 15 | 15 | 11 | < 0,25 |
| 60 | 42 | 15 | 21 | 23 | 17 | 3 |
| 120 | 23 | 24 | 13 | 4 | 5 | 7 |
| 180 | - | - | - | - | - | - |
| 240 | 12 | - | - | - | - | - |
| 360 | 3,8 | - | - | - | - | - |
| 480 | 0,25 | - | - | - | - | - |

1 CAPMUL MCM90: Ein Gemisch von Mono- und Diglyceriden gesättigter $C_8/C_{10}$-Fettsäuren mit 90% Monoglycerid.

2 Diacetin - Essigsäure-diglycerid.

3 Monoacetin - Essigsäure-monoglycerid.

4 Triacetin - Essigsäure-triglycerid.

5 Glyceryl-monolaurat ($C_{12}$)

6 CAPTEX 300 - Triglyceride mittlerer Kettenlänge (d.h. von gesättigten $C_8/C_{10}$-Fettsäuren). Warenzeichen von Stokely-Van Camp. Inc. Columbus, Ohio

Jodwert = 0,5
Farbe Lovibond Max. = 1.0 R
Säurewert = 0,1
Trübungspunkt max. = -5°C
Feuchtigkeit max. = 0,1%
Spezifisches Gewicht = 0,927
Viskosität = 23 cps bei 25°C

Tabelle 4

Blutspiegel an Ceftriaxon-Natriumsalz - Affen - µg/ml

| Verabreichungs-art | i.V. | Enteral | Enteral | Oral | Oral "enteric coated"-Kapsel[1] |
|---|---|---|---|---|---|
| Träger | $H_2O$ | CAPMUL 8210 | $H_2O$ | CAPMUL 8210 | |
| Zeit (Min.) | | | | | |
| 5 | 130 | 16 | - | - | - |
| 10 | 83 | 45 | - | - | - |
| 20 | 44 | 44 | 0 | 3,8 | - |
| 40 | 26 | 42 | - | - | - |
| 60 | 21 | 44 | 0 | 4,1 | 16 |
| 120 | 8 | 35 | 0 | 5,4 | 11 |
| 180 | - | - | - | 2,9 | 3 |
| 240 | - | - | 0 | 2,0 | <0,25 |
| 360 | - | - | 0 | 0 | 0 |

[1] Hartgelatinekapseln mit 20 mg Ceftriaxon in 100 µl CAPMUL 8210 und "enteric coated" - Durchschnitt aus drei Affen.

Die gleiche in vivo Versuchsführung und mikrobiologische in vitro Analyse wurde verwendet für die Untersuchung weiterer β-Lactam-Antibiotika in Kombination mit dem Potentiator CAPMUL 8210. Die Antibiotika und die Testorganismen sind nachstehend aufgeführt:

Cefotaxim - E. coli 136
Cephalexin - M. lutea ATCC 9341
Cefazolin - B. subtilis ATCC 6633
Cefamandol - M. lutea ATCC 9341
Cephradin - M. lutea ATCC 9341
Penicillin G - M. lutea ATCC 9341
Piperacillin - M. lutea ATCC 9341
Thienamycin - S. aureus ATCC 25923
Cefoxitin - E. coli 1346
Moxalactam - E. coli ATCC 10536
Cephalothin - B. subtilis ATCC 6633

Amoxicillin - M. lutea ATCC 9341
Mezlocillin - M. lutea ATCC 9341
Cefoperazon - K. pneumoniae A
Azthreonam - E. coli Leo - HA2M4
Amdinocillin - E. coli Leo - HA2

Die Ergebnisse als Blutspiegelwerte nach Verabreichung, an 250 g Ratten, von 20 mg des Antibiotikums (in Salzlösung oder Wasser oder mit dem Potentiator CAPMUL 8210) pro kg Körpergewicht sind in den anschliessenden Tabellen 5-20 festgehalten:

Tabelle 5

Blutspiegel an Cefoxitin - Ratten - µg/ml

| Verabreichungsart | i.V. | Enteral | Enteral |
|---|---|---|---|
| Träger | Salzlösung | CAPMUL 8210 | $H_2O$ |
| Zeit (Min.) | | | |
| 5 | 59,2 | 8,8 | 0,7 |
| 10 | 42,6 | 7,7 | 0,7 |
| 20 | 24,8 | 10,5 | 0,8 |
| 40 | 19,0 | 10,8 | 0,8 |
| 60 | 9,7 | 18,2 | 1,0 |
| 120 | 0,2 | 9,4 | 0,8 |
| 240 | 0,2 | 0,7 | 0,6 |
| 360 | 0,2 | 0,2 | 0,2 |

Tabelle 6

Blutspiegel an Thienamycin - Ratten - µg/ml

| Verabreichungs-art | i.V. | Enteral | Enteral |
|---|---|---|---|
| Träger | Salzlösung | CAPMUL 8210 | $H_2O$ |
| Zeit (Min.) | | | |
| 5 | 45,9 | 4,1 | 0,2 |
| 10 | 20,2 | 5,4 | 0,3 |
| 20 | 11,7 | 4,3 | N.D. |
| 40 | 5,2 | 2,7 | N.D. |
| 60 | 2,1 | 3,0 | N.D. |
| 120 | 0,2 | 0,7 | N.D. |
| 240 | N.D. | N.D. | N.D. |
| 360 | N.D. | N.D. | N.D. |

Tabelle 7

Blutspiegel an Azthreonam - Ratten - µg/ml

| Verabreichungs-art | i.V. | Enteral | Enteral |
|---|---|---|---|
| Träger | Salzlösung | CAPMUL 8210 | $H_2O$ |
| Zeit (Min.) | | | |
| 5 | 112 | 3,6 | 0,9 |
| 10 | 94,0 | 6,1 | 0,9 |
| 20 | 59,3 | 5,9 | 0,9 |
| 40 | 37,1 | 6,8 | 1,1 |
| 60 | 22,2 | 6,6 | 1,1 |
| 120 | 6,2 | 4,1 | 1,4 |
| 240 | N.D. | 0,9 | N.D. |
| 360 | N.D. | 0,9 | N.D. |

N.D. = nicht nachweisbar

Tabelle 8

Blutspiegel an Cephalexin - Ratten - µg/ml

| Verabreichungsart | i.V. | Enteral | Enteral | Oral |
|---|---|---|---|---|
| Träger | Salzlösung | CAPMUL 8210 | $H_2O$ | $H_2O$ |
| Zeit (Min.) | | | | |
| 5 | 40 | 2 | 1 | N.D. |
| 10 | 40 | 2 | 3 | N.D. |
| 20 | 19 | 8 | 10 | 4 |
| 40 | 11 | 9 | 5 | 6 |
| 60 | 4 | 7 | 13 | 5 |
| 120 | N.D. | 7 | 11 | 4 |

Table 9

Blutspiegel an Moxalactam - Ratten - µg/ml

| Verabreichungsart | i.V. | Enteral | Enteral |
|---|---|---|---|
| Träger | Salzlösung | CAPMUL 8210 | $H_2O$ |
| Zeit (Min.) | | | |
| 5 | 121 | 9,6 | N.D. |
| 10 | 181 | 14,0 | N.D. |
| 20 | 186 | 16,5 | N.D. |
| 40 | 84,0 | 17,9 | N.D. |
| 60 | 65,7 | 19,9 | N.D. |
| 120 | 19,9 | 17,6 | N.D. |
| 240 | 2,5 | 7,3 | N.D. |
| 360 | N.D. | 2,5 | N.D. |

N.D. = nicht nachweisbar

Tabelle 10

Blutspiegel an Cephradin - Ratten - µg/ml

| Verabreichungs-art | i.V. | Enteral | Enteral | Oral | Oral |
|---|---|---|---|---|---|
| Träger | Salzlösung | CAPMUL 8210 | $H_2O$ | CAPMUL 8210 | $H_2O$ |
| Zeit (Min.) | | | | | |
| 5 | 67,3 | 3,9 | N.D. | 2,4 | 1,1 |
| 10 | 45,1 | 6,5 | N.D. | 2,9 | 1,5 |
| 20 | 20,7 | 6,5 | 2,2 | 2,6 | 1,7 |
| 40 | 18,6 | 5,5 | 3,3 | 2,1 | 2,4 |
| 60 | 9,4 | 6,3 | 5,1 | 1,2 | 3,2 |
| 120 | 2,7 | 8,2 | 7,9 | 1,8 | 6,3 |
| 240 | 1,0 | 4,4 | 7,3 | 2,8 | 3,0 |
| 360 | N.D. | 2,3 | 2,8 | - | 1,7 |
| 480 | - | - | - | 1,6 | 1,0 |

\- = keine Probe
N.D. = nicht nachweisbar

Tabelle 11

Blutspiegel an Cephalothin - Ratten - µg/ml

| Verabreichungsart | i.V. | Enteral | Enteral |
|---|---|---|---|
| Träger | Salzlösung | CAPMUL 8210 | $H_2O$ |
| Zeit (Min.) | | | |
| 5 | 114 | 9,0 | N.D. |
| 10 | 105 | 11,2 | N.D. |
| 20 | 60,1 | 11,8 | N.D. |
| 40 | 36,8 | 10,3 | - |
| 60 | 10,4 | 6,0 | - |
| 120 | 1,9 | 2,4 | N.D. |
| 240 | N.D. | N.D. | N.D. |
| 360 | N.D. | N.D. | N.D. |

N.D. = nicht nachweisbar
- = keine Probe

Tabelle 12

Blutspiegel an Cefamandol - Ratten - µg/ml

| Verabreichungsart | i.V. | Enteral | Enteral |
|---|---|---|---|
| Träger | Salzlösung | CAPMUL 8210 | $H_2O$ |
| Zeit (Min.) | | | |
| 5 | 50,9 | 5,9 | N.D. |
| 10 | 42,5 | 9,9 | N.D. |
| 20 | 29,2 | 12,6 | N.D. |
| 40 | 18,3 | 11,4 | Spuren |
| 60 | 11,6 | 10,3 | 1,9 |
| 120 | 2,9 | 4,8 | 2,1 |
| 240 | N.D. | 1,8 | N.D. |

N.D. = nicht nachweisbar

## Tabelle 13

Blutspiegel an Cefotaxim - Ratten - µg/ml

| Verabreichungs-art | i.V. | Enteral | Enteral | Oral |
|---|---|---|---|---|
| Träger | Salzlösung | CAPMUL 8210 | $H_2O$ | CAPMUL 8210 |
| Zeit (Min.) | | | | |
| 5 | 104 | N.D. | N.D. | 2,3 |
| 10 | 80 | 23 | N.D. | 1,8 |
| 20 | 42 | 14 | N.D. | 1,7 |
| 40 | 25 | 13 | N.D. | 1,1 |
| 60 | 18 | 11 | N.D. | 0,79 |
| 120 | N.D. | 4 | N.D. | Spuren |

## Tabelle 14

Blutspiegel an Cefoperazon - Ratten - µg/ml

| Verabreichungs-art | i.V. | Enteral | Enteral |
|---|---|---|---|
| Träger | Salzlösung | CAPMUL 8210 | $H_2O$ |
| Zeit (Min.) | | | |
| 5 | 98,8 | 5,6 | N.D. |
| 10 | 66,9 | 6,1 | N.D. |
| 20 | 34,2 | 6,0 | N.D. |
| 40 | 16,0 | 7,9 | N.D. |
| 60 | 9,8 | 5,0 | N.D. |
| 120 | 1,4 | 2,5 | N.D. |
| 240 | N.D. | 0,8 | N.D. |
| 360 | N.D. | N.D. | N.D. |

N.D. = nicht nachweisbar

Tabelle 15

Blutspiegel an Mezlocillin - Ratten - µg/ml

| Verabreichungsart<br>Träger<br>Zeit (Min.) | i.V.<br>Salzlösung | Enteral<br>CAPMUL 8210 | Enteral<br>$H_2O$ |
|---|---|---|---|
| 5 | 47,3 | 3,9 | N.D. |
| 10 | 27,3 | 5,8 | N.D. |
| 20 | 18,2 | 6,2 | N.D. |
| 40 | 8,6 | 5,0 | N.D. |
| 60 | 6,0 | 3,4 | N.D. |
| 120 | 0,8 | 1,2 | N.D. |
| 240 | N.D. | N.D. | N.D. |
| 360 | N.D. | N.D. | N.D. |

Tabelle 16

Blutspiegel an Amoxicillin - Ratten - µg/ml

| Verabreichungsart<br>Träger<br>Zeit (Min.) | i.V.<br>Salzlösung | Enteral<br>CAPMUL 8210 | Enteral<br>$H_2O$ | Oral<br>$H_2O$ |
|---|---|---|---|---|
| 5 | 40,4 | 1,8 | 0,4 | 0,4 |
| 10 | 26,4 | 3,0 | 0,5 | 3,4 |
| 20 | 16,8 | 3,2 | 1,0 | 0,8 |
| 40 | 11,3 | 3,2 | 1,4 | 1,7 |
| 60 | 5,9 | 2,9 | 1,8 | 2,4 |
| 120 | 2,8 | - | 2,6 | 2,1 |
| 240 | 0,5 | 1,5 | 2,0 | 2,7 |
| 360 | 0,2 | 0,9 | 1,4 | 0,8 |

N.D. = nicht nachweisbar

## Tabelle 17

Blutspiegel an Piperacillin - Ratten - µg/ml

| Verabreichungs-art | i.V. | Enteral | Enteral |
|---|---|---|---|
| Träger | Salzlösung | CAPMUL 8210 | $H_2O$ |
| Zeit (Min.) | | | |
| 5 | 49,9 | 1,3 | N.D. |
| 10 | 28,7 | 1,4 | N.D. |
| 20 | 11,9 | 1,7 | N.D. |
| 40 | 8,5 | 1,4 | N.D. |
| 60 | 2,9 | 0,8 | N.D. |
| 120 | 1,3 | N.D. | N.D. |
| 240 | N.D. | N.D. | N.D. |
| 360 | 2,8 | N.D. | N.D. |

## Tabelle 18

Blutspiegel an Cefazolin - Ratten - µg/ml

| Verabreichungs-art | i.V. | Enteral | Enteral |
|---|---|---|---|
| Träger | Salzlösung | CAPMUL 8210 | $H_2O$ |
| Zeit (Min.) | | | |
| 5 | 105 | 8 | N.D. |
| 10 | 91 | 13 | N.D. |
| 20 | 81 | 21 | N.D. |
| 40 | 50 | 14 | N.D. |
| 60 | 32 | 11 | N.D. |
| 120 | N.D. | N.D. | N.D. |

N.D. = nicht nachweisbar

Tabelle 19

Blutspiegel an Amdinocillin - Ratten - µg/ml

| Verabreichungsart | i.V. | Enteral | Enteral |
|---|---|---|---|
| Träger | Salzlösung | CAPMUL 8210 | $H_2O$ |
| Zeit (Min.) | | | |
| 5 | 65,5 | 3,0 | N.D. |
| 10 | 47,7 | 3,5 | 0,3 |
| 20 | 24,3 | 4,2 | 0,3 |
| 40 | 10,0 | 5,3 | 0,3 |
| 60 | 5,4 | 6,0 | 0,3 |
| 120 | 1,0 | 4,1 | N.D. |
| 240 | N.D. | 0,8 | N.D. |
| 360 | N.D. | 0,3 | N.D. |

N.D. = nicht nachweisbar

Tabelle 20

Blutspiegel an Penicillin G - Ratten - µg/ml

| Verabreichungs-art | i.V. | Enteral | Enteral | Subkutan | Oral | Oral |
|---|---|---|---|---|---|---|
| Träger | Salzlösung | CAPMUL 8210 | $H_2O$ | $H_2O$ | CAPMUL 8210 | $H_2O$ |
| Zeit (Min.) | | | | | | |
| 5 | 74,2 | 7,0 | 0,6 | 4,9 | - | - |
| 10 | 45,5 | 7,8 | 0,5 | 11,1 | 4,1 | 2,5 |
| 20 | 18,6 | 9,5 | 0,7 | 15,9 | 7,4 | 2,2 |
| 40 | 9,9 | 10,4 | 0,9 | 9,6 | 3,4 | 3,4 |
| 60 | 3,2 | 10,5 | 1,5 | 4,1 | 2,5 | 2,3 |
| 120 | 0,4 | 6,2 | 1,6 | 0,5 | 0,5 | 1,0 |
| 240 | N.D. | 2,5 | 1,1 | N.D. | N.D. | 0,7 |
| 360 | N.D. | 0,4 | 0,8 | N.D. | N.D. | N.D. |

N.D. = nicht nachweisbar
- = keine Probe

Wie oben erläutert erfolgte keine Erhöhung der Plasmaspiegel bei Verwendung des Potentiators mit Cephalexin und Cephradin, zwei an sich oral wirksamen Cephalosporinen, oder mit Amoxicillin, einem oral wirksamen Penicillin. Verstärkung der intestinalen (enteralen) Absorption erfolgte bei Verwendung von CAPMUL 8210 oder CAPMUL MCM90 mit den anderen, an sich oral inaktiven β-Lactam-Antibiotika. Aehnlich wie für Ceftriaxon zeigten diese β-Lactam-Antibiotika erhöhte enterale Absorption mit CAPMUL 8210 oder ähnlichen Trägern. Diese Kombinationen können, durch ein "enteric coating" geschützt, auch oral verabreicht werden.

Für die Verwendung in der Humanmedizin werden Dosierungsformen im Bereich von etwa 25 bis etwa 2000 mg, vorzugsweise 50 bis etwa 500 mg an β-Lactam-Antibiotikum mit dem Potentiator/Träger verwendet.

<u>In vitro Versuch</u>

Ein in vitro-Modell zur Ermittlung von Transportcharakteristika und dadurch Identifikation von verwendbaren Trägern für die systemische Abgabe der β-Lactam-Antibiotika besteht aus einer Zelle zur Messung der Durchlässigkeit einer synthetischen Membran in verschiedenen Trägern und bei verschiedenen Konzentrationen des Antibiotikums. Das verwendete Modell wird beschrieben in Behl et al., Journal of Investigative Dermatology <u>75</u>, Seiten 346-352, 1980 und Durrheim et al., Journal of Pharmaceutical Sciences <u>69</u>, Nr. 7, Seiten 781-786, 1980. Als synthetische Membran wird eine Siloxan-Flüssigmembran verwendet. Die Zelle besteht aus einem Behälter mit zwei Abteilen und der Membran dazwischen. Das Empfängerabteil ("receiving compartment") enthält ein pH 7,4 Buffer (Clarks-Subs buffer) und das Spenderabteil ("donor compartment") enthält den ausgewählten Potentiator/Träger, d.h. CAPMUL 8210 oder Diacetin, etc., mit einem $C_1$-$C_8$-Alkanol als die Lipophilie erhöhendes Agens. Radio-markiertes β-Lactam-Antibiotikum wird im

Donorabteil vorgelegt. Bei Zeit Null werden in bestimmten Zeitabständen Proben aus dem Empfängerabteil genommen. Die gemessene Antibiotikamenge im Empfängerabteil wird als Funktion der Zeit in einem Diagramm aufgeführt. Die Kurve ist linear und dient zur Berechnung des Durchlässigkeitskoeffizientes (P-Wert) gemäss Ficks erstem Diffusionsgesetz. Eine Puffer/Puffer-Kontrolle wird zu Vergleichszwecken eingesetzt. Die Effektivität des Trägers ist eine Funktion des P-Wertes, d.h. ein P-Wert grösser als derjenige der Puffer/Puffer-Kontrolle zeigt Absorptionserhöhung an.

Die Durchlässigkeitskoeffizienten verschiedener Potentiatoren/Träger als 50% Gemisch mit CAPMUL 8210 sind wie folgt:

| Träger (50% CAPMUL 8210 und 50% absorptionserhöhender Träger) | $P \times 10^6$ (cm/Std.)* |
|---|---|
| Adjuvans | |
| Monoacetin | 47,20 |
| Diacetin | 27,10 |
| Triacetin | 15,80 |
| CAPMUL 8210 | 9,10 |
| CAPTEX 300 | 20,50 |
| Kontrolle | 3,3 |

* anfängliche Steigung

Wie oben erwähnt erfolgt die orale Verabreichung des β-Lactam-Antibiotikum/Potentiator-Gemisches mit Hilfe einer festen "enteric coated"-Dosierungsform, um das β-Lactam-Antibiotikum vor der Magensäure zu schützen. Die dafür verwendeten Träger sind vornehmlich in flüssiger Form, können jedoch in fester Form vorliegen. Die Gemische können in Hart- oder Weichkapseln gefüllt oder der flüssige Träger kann an einem festen Träger adsorbiert werden, beispielsweise an Stärke zwecks Erhalts eines fliessfähigen Pulvers und anschliessend in die erwähnten Kapseln abgefüllt oder zu Tabletten mit geeignetem "enteric coating" komprimiert werden.

Andere Dosierungsformen können "enteric coated" bzw. nicht-"enteric coated" Systeme beinhalten, d.h. Kapseln oder Tabletten, worin das β-Lactam-Antibiotikum und der Potentiator selbst "enteric coated" sind. Das "enteric coating" des einzelnen β-Lactam-Antibiotikums und/oder Potentiators kann durch Verwendung von mikro-gekapselten Formen des β-Lactams und/oder Potentiators zwecks Erhalts eines fliessfähigen Pulvers für die Abfüllung in Hart- oder Weichkapseln oder zur Komprimierung als Tabletten bewerkstelligt werden. Eine mögliche Ausführungsform ist eine "enteric coated" Mikrokapsel oder Beadlet-form des β-Lactam-Antibiotikums entweder allein oder als Suspension in einem flüssigen Potentiator, welches Gemisch anschliessend in eine "enteric coated" oder nicht-"enteric coated" Kapsel eingekapselt werden kann.

Die erwähnten Präparate sind in "enteric coating" Materialien eingehüllt, damit das β-Lactam-Antibiotikum vor dem Magensaft geschützt ist und die optimale Abgabe der β-Lactam/Potentiator-Kombination im Darm gewährleistet wird. Das "enteric coating" Material wird durch den Magensaft nicht beeinflusst, wird jedoch im Darmsaft aufgelöst, wobei der aktive Wirkstoff freigesetzt wird. Die Wirksamkeit solcher "enteric coated" Produkte kann anhand bekannter USP-Methoden ermittelt werden.

Als "enteric-coating" Materialien kommen z.B. in Frage:

- Celluloseacetatphthalat
- Hydroxypropylmethylcellulosephthalat
- Polyvinylacetatphthalat
- Shellac
- Methacrylsäure und Methacrylsäureester
- Zein
- Andere dem Fachmann bekannten Materialien.

Diese "enteric coating" Materialien können mit oder ohne Weichmacher, wie acetylierte Glyceride, Diäthylphthalat usw., eingesetzt werden, unter Verwendung von dem Fachmann bekannten Methoden.

Beispiele von "enteric coating" Lösungen sind:

A

| | |
|---|---|
| - Hydroxypropylmethylcellulosephthalat (HPMCP) | 5,0 |
| - Triacetin | 0,5 |
| - Methylenchlorid | 47,25 |
| - Denaturierter Alkohol | 47,25 |

B

| | |
|---|---|
| - HPMCP | 10,0 |
| - Titandioxid | 0,2 |
| - Dimethylpolysiloxan | 0,05 |
| - Aceton | 44,875 |
| - Denaturierter Alkohol | 44,875 |

C

| | |
|---|---|
| - Celluloseacetatphthalat (CAP) | 8,5 |
| - Diäthylphthalat | 1,5 |
| - Titandioxid | 0,2 |
| - Aceton | 44,9 |
| - Denaturierter Alkohol | 44,9 |

D

| | |
|---|---|
| - Polyvinylacetatphthalat | 5,0 |
| - Acetylierte Glyceride | 0,8 |
| - Methylenchlorid | 47,1 |
| - Denaturierter Alkohol | 47,1 |

E

| | |
|---|---|
| - Eudragit S oder L* | 8 |
| - Aceton | 46 |
| - Wasserfreier Alkohol | 46 |
| - Weichmacher | q.s. |

* Eudragit S oder L sind Markennamen (Röhm. Pharma., GMBH, Weiterstadt, Deutschland) für Methacrylsäure oder Methacrylsäureester.

Das "enteric coating" wird zu etwa 1-10%, vorzugsweise 2-8% des Kapsel- bzw. Tablettengewichts appliziert.

In den Zubereitungsformen werden β-Lactam-Antibiotikum/Potentiator-Verhältnisse zwischen etwa 1:32 bis etwa 1:0,5, vorzugsweise 1:16 bis 1:3, insbesondere etwa 1:8, verwendet. Wie oben erwähnt werden bedeutende Antibiotikaspiegel an der Ratte bei einem Antibiotikum/Potentiator-Verhältnis bis etwa 1:100 erreicht.

Die erfindungsgemässen Präparate können ebenfalls an sich bekannte Hilfsstoffe zwecks Erreichung einer gewünschten Konsistenz enthalten.

Die erfindungsgemässen Präparate können feste oder flüssige Formulierungen für die orale Verabreichung darstellen. Beispielsweise können sie in der Form gewöhnlicher Kapseln oder "enteric coated" Kapseln, Mikrokapseln oder Beadlets sein oder Tabletten, wie z.B. "enteric coated" Tabletten oder Tabletten mit einem "enteric coated" mikro-eingekapselten Gemisch von Potentiator und β-Lactam-Antibiotikum, oder die beiden Komponenten können je für sich "enteric coated" sein. Diese Zubereitungsformen können zusätzlich herkömmliche pharmazeutische Träger und Zusätze enthalten, insbesondere die Viskosität verbessernde und/oder Struktur- oder Matrix-bildende Zusätze zwecks Erhalts geeigneter Viskosität und physikalischer Struktur. Geeignete Zusätze sind z.B. Verdickungsmittel, wie z.B. hochdisperse Kieselsäure (z.B. die kommerziellen Produkte "37 Aerosil"), Bentonite, kolloidaler Ton, Carboxymethylcellulosen, modifizierte Montmorillonite, z.B. Alkylammoniumsalze von Montmorilloniten (z.B. die kommerziellen Produkte "Bentone"), worin die Alkylgruppe 16-18 Kohlenstoffatome enthalten, organische Verdickungs- und Struktur-bildende Agenzien,

wie gesättigte höhere Fettsäuren und Alkohole mit z.B. 12-20 Kohlenstoffatomen, wie z.B. Stearin- oder Palmitinsäure, Stearyl- oder Cetylalkohol, Wachse, wie Bienenwachs, synthetische Ester von höheren Fettsäuren und höheren Fettalkoholen, Walratfett, Monoglyceride von gesättigten oder ungesättigten höheren Fettsäuren, z.B. Monoglyceride von Stearinsäure, Palmitinsäure oder Oelsäure, partielle Glyceride von Polyhydroxyfettsäuren (z.B. die kommerziellen Produkte "Softigen 701"), gel-bildende Agenzien, wie z.B. Aluminiumstearat, Dispersionsmittel, wie anionische, nichtionische und kationische Schaumerzeuger, Emulgiermittel, wie Lecithin, und ähnliche Salze. Die Präparate können ferner pharmazeutische Adjuvantien enthalten, z.B. Bindungsmittel oder Gleitmittel für die Tablettierung, Stabilisierungsmittel, z.B. EDTA, Antioxidantien, z.B. Ascorbinsäure, Geschmacksmittel, Konservierungsmittel, z.B. Methyl- oder Propylparaben, und Puffer, z.B. Phosphate. Verwendbare Farbstoffe sind z.B. die akzeptierbaren "Food, Drug or Cosmetic" Farbstoffe. Als Trübungsmittel kann man z.B. Titandioxid verwenden.

Patentansprüche

1. Absorbierbares pharmazeutisches Präparat für enterale Verabreichung, dadurch gekennzeichnet, dass es eine therapeutisch wirksame Menge eines im wesentlichen oral inaktiven β-Lactam-Antibiotikums in einer absorptionserhöhenden Menge eines Potentiators enthält, der aus einer aliphatischen $C_2$-$C_{18}$-Fettsäure, einem Mono-, Di- oder Triglycerid einer $C_2$-$C_{12}$-Fettsäure, einem partiellen Ester oder Vollester von Propylenglykol, Polyäthylenglykol oder einem Kohlenhydrat mit einer $C_2$-$C_{12}$-Fettsäure, einem pharmazeutisch verträglichen Ester bzw. Aether davon oder einem Gemisch der erwähnten Potentiatoren besteht; erwünschtenfalls in Verbindung mit einem pharmazeutisch verträglichen Träger.

2. Oral wirksames Präparat nach Anspruch 1, dadurch gekennzeichnet, dass es in "enteric coated" Form vorliegt.

3. Präparat nach Anspruch 1 oder 2, dadurch gekennzeichnet, dass es eine Lösung oder Suspension des β-Lactam-Antibiotikums in der Fettsäure bzw. im Glycerid bzw. im Gemisch davon darstellt.

4. Präparat nach Anspruch 1 oder 2, dadurch gekennzeichnet, dass es ein in fester Form vorliegendes Gemisch des β-Lactam-Antibiotikums mit der Fettsäure bzw. dem Glycerid bzw. dem Gemisch davon darstellt.

5. Präparat nach einem der Ansprüche 1-4, dadurch gekennzeichnet, dass der Potentiator ein Glycerid oder ein Gemisch von Mono- und Diglyceriden einer Fettsäure darstellt, wobei die Monoglyceridkomponente dem Diglyceridanteil überwiegt.

6. Präparat nach einem der Ansprüche 1-4 in dosierter Form, dadurch gekennzeichnet, dass das β-Lactam-Antibiotikum zu etwa 25 mg bis etwa 2000 mg vorliegt.

7. Präparat nach Anspruch 6, dadurch gekennzeichnet, dass das β-Lactam-Antibiotikum zu etwa 50 bis etwa 500 mg vorliegt.

8. Präparat nach einem der Ansprüche 1-7, dadurch gekennzeichnet, dass der Potentiator ein Gemisch von Glyceriden von $C_8$-$C_{12}$-Fettsäuren darstellt.

9. Präparat nach einem der Ansprüche 1-8, dadurch gekennzeichnet, dass der Potentiator ein Glyceridgemisch in einer Menge zwischen etwa 33 Gew.% und etwa 98 Gew.% des Präparates darstellt.

10. Präparat nach Anspruch 9, dadurch gekennzeichnet, dass der Glyceridgemischanteil bei etwa 89 Gew.% des Präparates liegt.

11. Präparat nach einem der Ansprüche 1-10, dadurch gekennzeichnet, dass es in dosierter Form als "enteric coated" Kapseln, Mikrokapseln oder Beadlets vorliegt.

12. Präparat nach einem der Ansprüche 1, 2 und 4-10, dadurch gekennzeichnet, dass es in dosierter Form als Tabletten vorliegt.

13. Präparat nach einem der Ansprüche 1-12, dadurch gekennzeichnet, dass der Potentiator ein Gemisch von Mono- und Diglyceriden von $C_8/C_{10}$ gesättigten Fettsäuren darstellt, worin das Monoglycerid zu etwa 70% oder etwa 90% des Mono/Diglyceridgemisches vorliegt.

14. Präparat nach einem der Ansprüche 1-13, dadurch gekennzeichnet, dass das β-Lactam-Antibiotikum Ceftriaxon ist.

15. Präparat nach einem der Ansprüche 1-13, dadurch gekennzeichnet, dass das β-Lactam-Antibiotikum Cefoxitin, Thienamycin, Azthreonam, Moxalactam, Cephalothin, Cefamandol, Cefotaxim, Cefoperazon, Mezlocillin, Piperacillin, Cefazolin, Amdinocillin oder Penicillin G ist.

16. Präparat nach einem der Ansprüche 1-14, dadurch gekennzeichnet, dass das β-Lactam-Antibiotikum Ceftriaxon ist und der Potentiator ein Gemisch von Mono- und Diglyceriden von $C_8/C_{10}$-Fettsäuren darstellt, worin das Monoglycerid zu etwa 70% oder etwa 90% des Mono/Diglyceridgemisches vorliegt.

17. Verfahren zur Ueberführung von im wesentlichen oral inaktiven β-Lactam-Antibiotika in enteral wirksame Produkte, dadurch gekennzeichnet, dass man das erwähnte Antibiotikum mit einer absorptionserhöhenden Menge eines Potentiators kombiniert, der aus einer aliphatischen $C_2$-$C_{18}$-Fettsäure, einer Mono-, Di- oder Triglycerid eines $C_2$-$C_{12}$-Fettsäure, einem partiellen oder totalen Ester von Propylenglykol, Polyäthylenglykol oder einem Kohlenhydrat mit einer $C_2$-$C_{12}$-Fettsäure, einem pharmazeutisch verträglichen Ester bzw. Aether davon oder einem Gemisch der erwähnten Potentiatoren besteht; und erwünschtenfalls das Kombinationsprodukt mit einem pharmazeutisch verträglichen Träger vereinigt.

18. Verfahren nach Anspruch 17, dadurch gekennzeichnet, dass man durch Umwandlung des Kombinationsprodukts in "enteric coated" Form in ein oral wirksames Produkt überführt.

***

Patentansprüche für Oesterreich

1. Verfahren zur Ueberführung von im wesentlichen oral inaktiven β-Lactam-Antibiotika in enteral wirksame Produkte, dadurch gekennzeichnet, dass man das erwähnte Antibiotikum mit einer absorptionserhöhenden Menge eines Potentiators kombiniert, der aus einer aliphatischen $C_2$-$C_{18}$-Fettsäure, einem Mono-, Di- oder Triglycerid einer $C_2$-$C_{12}$-Fettsäure, einem partiellen Ester oder Vollester von Propylenglykol, Polyäthylenglykol oder einem Kohlenhydrat mit einer $C_2$-$C_{12}$-Fettsäure, einem pharmazeutisch verträglichen Ester bzw. Aether davon oder einem Gemisch der erwähnten Potentiatoren besteht; und erwünschtenfalls das Kombinationsprodukt mit einem pharmazeutisch verträglichen Träger vereinigt.

2. Verfahren nach Anspruch 1, dadurch gekennzeichnet, dass man durch Umwandlung des Kombinationsprodukts in "enteric coated" Form in ein oral wirksames Produkt überführt.

3. Verfahren nach Anspruch 1 oder 2, dadurch gekennzeichnet, dass man das β-Lactam-Antibiotikum und die Fettsäure bzw. das Glycerid bzw. das Gemisch davon in einer Lösung oder Suspension kombiniert.

4. Verfahren nach Anspruch 1 oder 2, dadurch gekennzeichnet, dass man das β-Lactam-Antibiotikum und die Fettsäure bzw. das Glycerid bzw. das Gemisch davon in einem in fester Form vorliegenden Gemisch kombiniert.

5. Verfahren nach einem der Ansprüche 1-4, dadurch gekennzeichnet, dass man einen Potentiator verwendet, der aus einem Glycerid oder einem Gemisch von Mono- und Diglyceriden einer Fettsäure besteht, wobei die Monoglyceridkomponente dem Diglyceridanteil überwiegt.

6. Verfahren nach einem der Ansprüche 1-4, dadurch gekennzeichnet, dass man das Produkt in dosierter Form herstellt, worin das β-Lactam-Antibiotikum zu etwa 25 mg bis etwa 2000 mg vorliegt.

7. Verfahren nach Anspruch 6, dadurch gekennzeichnet, dass das β-Lactam-Antibiotikum zu etwa 50 bis etwa 500 mg vorliegt.

8. Verfahren nach einem der Ansprüche 1-7, dadurch gekennzeichnet, dass man einen Potentiator verwendet, der ein Gemisch von Glyceriden von $C_8$-$C_{12}$-Fettsäuren darstellt.

9. Verfahren nach einem der Ansprüche 1-8, dadurch gekennzeichnet, dass man einen Potentiator verwendet, der ein Glyceridgemisch in einer Menge zwischen etwa 33 Gew.% und etwa 98 Gew.% des Präparates darstellt.

10. Verfahren nach Anspruch 9, dadurch gekennzeichnet, dass der Glyceridgemischanteil bei etwa 89 Gew.% des Präparates liegt.

11. Verfahren nach einem der Ansprüche 1-10, dadurch gekennzeichnet, dass man das Produkt in dosierter Form als "enteric coated" Kapseln, Mikrokapseln oder Beadlets herstellt.

12. Verfahren nach einem der Ansprüche 1, 2 und 4-10, dadurch gekennzeichnet, dass man das Produkt in dosierter Form als Tabletten herstellt.

13. Verfahren nach einem der Ansprüche 1-12, dadurch gekennzeichnet, dass man einen Potentiator verwendet, der ein Gemisch von Mono- und Diglyceriden von $C_8/C_{10}$ gesättigten Fettsäuren darstellt, worin das Monoglycerid zu etwa 70% oder etwa 90% des Mono/Diglyceridgemisches vorliegt.

14. Verfahren nach einem der Ansprüche 1-13, dadurch gekennzeichnet, dass man als β-Lactam-Antibiotikum Ceftriaxon verwendet.

15. Verfahren nach einem der Ansprüche 1-13, dadurch gekennzeichnet, dass man als β-Lactam-Antibiotikum Cefoxitin, Thienamycin, Azthreonam, Moxalactam, Cephalothin, Cefamandol, Cefotaxim, Cefoperazon, Mezlocillin, Piperacillin, Cefazolin, Amdinocillin oder Penicillin G verwendet.

16. Verfahren nach einem der Ansprüche 1-14, dadurch gekennzeichnet, dass man als β-Lactam-Antibiotikum Ceftriaxon und als Potentiator ein Gemisch von Mono- und Diglyceriden von $C_8/C_{10}$ gesättigten Fettsäuren verwendet, worin das Monoglycerid zu etwa 70% oder etwa 90% des Mono/Diglyceridgemisches vorliegt.

***

0108295

Europäisches Patentamt

# EUROPÄISCHER RECHERCHENBERICHT

Nummer der Anmeldung

EP 83 11 0343

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| D,X Y | BE-A- 567 598 (NOVO TERAPEUTISK LABORATORIUM A/S) * Seite 1, Absatz 2; Seite 4, Zeile 14-30; Seite 6, Zeilen 9-17; Ansprüche * --- | 1,3-10,12-17 | A 61 K 9/00<br>A 61 K 47/00<br>A 61 K 31/43<br>A 61 K 31/545<br>A 61 K 31/40<br>A 61 K 31/535 |
| X,Y | CHEMICAL ABSTRACTS, Band 86, Nr. 12, 21. März 1977, Seite 396, Nr. 78631b, Columbus, Ohio, US K.H. BAUER u.a.: "Urine recovery studies after the delivery of ampicillin in aqueous and oily fluid bases" & PHARM. IND. 1976, 38(11), 987-8 * Zusammenfassung * --- | 1,6,7,17 | |
| X,Y | US-A-4 273 763 (D.F. HORROBIN) * Spalte 3, Zeilen 8-28; Spalte 3, Zeile 62 - Spalte 4, Zeile 16; Spalte 6, Zeile 44 - Spalte 7, Zeile 11; Beispiele; Ansprüche * --- | 1,3-10,12-17 | RECHERCHIERTE SACHGEBIETE (Int. Cl. 3) |
| X,Y | US-A-4 079 138 (SONG-LIG LIN) * Spalte 2, Zeilen 37-50; Spalte 3, Zeilen 10-22; Anspruch 1 * --- | 1,3-10,12,17 | A 61 K 9/00<br>A 61 K 47/00<br>A 61 K 31/00 |
| X,Y | US-A-4 079 131 (SONG-LING LIN) * Spalte 2, Zeilen 37-50; Spalte 3, Zeilen 10-22; Anspruch 1 * --- -/- | 1,3-10,12-17 | |

Der vorliegende Recherchenbericht wurde für alle Patentansprüche erstellt.

| Recherchenort | Abschlußdatum der Recherche | Prüfer |
|---|---|---|
| DEN HAAG | 17-01-1984 | BERTE M.J. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82

0108295

Europäisches Patentamt

# EUROPÄISCHER RECHERCHENBERICHT

Nummer der Anmeldung

EP 83 11 0343

## EINSCHLÄGIGE DOKUMENTE

Seite 2

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| X,Y | CHEMICAL ABSTRACTS, Band 73, Nr. 2, 13. Juli 1970, Seite 253, Nr. 7218t, Columbus, Ohio, US & ZA - A - 68 05 560 (BRISTOL-MYERS CO.) 29-08-1969 * Zusammenfassung * --- | 1,2,11,12,18 | |
| Y | FR-A-2 350 836 (SHIONOGI CO. LTD.) * Seite 1, Zeilen 26-30; Seite 17, Beispiele; Ansprüche * --- | 1,2,11,12,18 | |
| D,Y | FR-A-2 206 943 (SUMITOMO) * Seite 4, Zeilen 7-33; Seite 3, Zeilen 20-32; Ansprüche 1,3,4,8,9,12 * --- | 1,3-5,8-10,13,16,17 | |
| Y | GB-A-2 015 339 (SANDOZ LTD.) * Spalte 1, Zeile 111 - Spalte 2, Zeile 126; Anspruch 1 * --- | 1,3-10,12-17 | RECHERCHIERTE SACHGEBIETE (Int. Cl. 3) |
| Y | CHEMICAL ABSTRACTS, Band 97, Nr. 18, November 1982, Seiten 890, Nr. 150678u, Columbus, Ohio, US J.P. DECHESNE u.a.: "Study on the effect of plasticizers on properties of applied films of EUDRAGIT L 30 D" & J. PAHRM. BELG. 1982, 37(4), 283-6 * Zusammenfassung * --- -/- | 2,11,12,18 | |

Der vorliegende Recherchenbericht wurde für alle Patentansprüche erstellt.

| Recherchenort | Abschlußdatum der Recherche | Prüfer |
|---|---|---|
| DEN HAAG | 17-01-1984 | BERTE M.J. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82

Europäisches Patentamt

# EUROPÄISCHER RECHERCHENBERICHT

Nummer der Anmeldung

EP 83 11 0343

Seite 3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| Y | FR-A-2 320 749 (BEECHAM GROUP LTD.)<br>* Seite 2, Zeilen 8-29; Seite 3, Zeile 37 - Seite 4, Zeile 11; Ansprüche *<br>----- | 1,3-10,12-17 | |

RECHERCHIERTE SACHGEBIETE (Int. Cl. 3)

Der vorliegende Recherchenbericht wurde für alle Patentansprüche erstellt.

| Recherchenort | Abschlußdatum der Recherche | Prüfer |
|---|---|---|
| DEN HAAG | 17-01-1984 | BERTE M.J. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82